# EUROPÄISCHE PATENTSCHRIFT

(11) **EP 4 658 334 B1**
(45) Veröffentlichungstag und Bekanntmachung des Hinweises auf die Patenterteilung: **21.10.2026**
(21) Anmeldenummer: 24703137.0
(22) Anmeldetag: 31.01.2024
(51) Int. Cl.: A61M 1/16, A61J 1/10, A61J 1/20

(54) **MEHRKAMMERBEUTEL ZUR HERSTELLUNG VON DIALYSAT**
MULTI-CHAMBER BAG FOR THE PRODUCTION OF DIALYSATE
SAC À PLUSIEURS CHAMBRES POUR LA PRODUCTION DE DIALYSAT

(30) Priorität: 02.02.2023 DE 102023102598
(43) Veröffentlichungstag der Anmeldung: 10.12.2025
(73) Patentinhaber: Fresenius Medical Care Deutschland GmbH, 61352 Bad Homburg (DE)
(72) Erfinder: GASTAUER, Paul, 61352 Bad Homburg (DE); KUGELMANN, Franz, 61352 Bad Homburg (DE); SCHLEY, Christian, 61352 Bad Homburg (DE); JOACHIMSKY, Pascal, 61352 Bad Homburg (DE)
(74) Vertreter: Lorenz Seidler Gossel Part. mbB
(86) Internationale Anmeldenummer: PCT/EP2024/052298
(87) Internationale Veröffentlichungsnummer: WO 2024/160861

(56) Entgegenhaltungen:
- EP-B1- 1 101 483
- EP-B1- 1 561 448
- DE-A1- 102018 116 787

## Beschreibung

Die vorliegende Erfindung betrifft einen Mehrkammerbeutel zur Herstellung von Dialysat sowie ein Verfahren zur Herstellung eines derartigen Mehrkammerbeutels.

Bei der Dialyse kommen häufig Beutel zum Einsatz, die vorgefertigtes Dialysat oder zumindest Teilkomponenten davon, enthalten. Beispielsweise kann es aus Gründen der Lagerungsstabilität vorteilhaft sein, Teilkomponenten einer Lösung, beispielsweise Dialysats, separat zu lagern und erst unmittelbar vor der Verwendung der Lösung zu vermischen.

Hierfür kommen häufig Mehrkammerbeutel zum Einsatz, welche in separaten Kammern separate Teilkomponenten enthalten, welche unmittelbar vor der Verwendung einer erzeugten Lösung miteinander vermischt werden.

Beispielsweise könnend derartige Mehrkammerbeutel zwei verschiedene Lösungen, eine Lösung und ein Trockenkonzentrat, eine Lösung und ein pastöses Konzentrat oder sonstige beliebige Inhalte aufweisen. Beispiele hierfür sind Lösungen zur Peritonealdialyse oder zur Hemofiltration sowie Zubereitungen zur parenteralen Ernährung.

EP 0 935 967 offenbart eine Lösung für die Peritonealdialyse bestehend aus zwei Einzellösungen, die in einem Zweikammerbeutel bereitgestellt wird. Die erste Einzellösung umfasst Calciumionen, weitere Elektrolytsalze und Glucose in osmotisch wirksamer Konzentration. Die zweite Einzellösung umfasst Bicarbonat und das Salz einer schwachen Säure mit pka 〈 5. Vor Gebrauch müssen beide Kammern gemischt werden, um die gebrauchsfertige Peritonealdialyselösung zu erhalten.

WO 2003/059417 offenbart eine zweiteilige Dialyselösung, umfassend eine erste Komponente, umfassend ein Bicarbonatkonzentrat, eine zweite Komponente, umfassend ein Elektrolytkonzentrat, die in einem Zweikammerbeutel bereitgestellt wird und unmittelbar vor Gebrauch gemischt wird.

Die DE 10 2018 116787 A1 offfenbart einen Mehrkammerbeutel für medizinische Zwecke, der von aufeinander angeordneten Folien, insbesondere flexiblen Folien, aus einem zumindest teilweise lichtdurchlässigen Material gebildet wird, die mit mindestens einer nicht auftrennbaren Naht flüssigkeitsdicht miteinander verbunden sind, wobei der Beutel von mindestens einer auftrennbaren Naht, mit der die aufeinander angeordneten Folien flüssigkeitsdicht miteinander verbunden sind, in mindestens zwei Kammern unterteilt ist.

Die EP 1 101 483 B1 offenbart einen Behälter zur Bereitstellung eines medizinischen Konzentrates, insbesondere für die Nierendialyse, bestehend aus mindestens zwei Kompartimenten und lösbaren Trennmitteln zwischen den Kompartimenten, wobei das erste Kompartiment mit einem ersten im wesentlichen glucosehaltigen Teilkonzentrat gefüllt ist, dessen Glucosegehalt über 10% liegt, und das zweite Kompartiment ein zweites Teilkonzentrat mit den Elektrolyten Calcium, Magnesium und Kalium in saurer Lösung enthält, wobei das elektrolythaltige Konzentrat freie Salzsäure enthält.

Die EP 1 561 448 B1 offenbart einen Mehrkammerbehälter, der anzeigen kann, ob eine abziehbare Trennwand abgezogen ist oder nicht, sodass ein medizinischer

Fachmann bei Verwendung des Mehrkammerbehälters schnell einen Überblick über den Zustand der Verbindung zwischen den jeweiligen Kammern erhalten kann, wobei der Mehrkammerbehälter aus einer Kunststofffolie und mehreren Kammern besteht, die durch eine abziehbare Trennwand unterteilt sind, die durch thermisches Verschweißen eines Teils der gegenüberliegenden Folien gebildet wird, dadurch gekennzeichnet, dass eine Einrichtung, die das Abziehen der Trennwand anzeigen kann, auf einer Oberfläche des Mehrkammerbehälters vorgesehen ist.

Herkömmliche Mehrkammerbeutel weisen zum Befüllen der jeweiligen Kammern jeweils einen Anschluss oder Befüllport auf, mittels welchem eine jede Kammer befüllbar ist. Die hierfür erforderlichen Konnektoren, Befüllstutzen und Verschlusskappen verkomplizieren die Fertigung und Anwendung derartiger Mehrkammerbeutel.

Vor diesem Hintergrund liegt der vorliegenden Erfindung die Aufgabe zugrunde, die Probleme des Stands der Technik abzumildern oder gar ganz zu beseitigen. Insbesondere liegt der vorliegenden Erfindung die Aufgabe zugrunde, einen vereinfachten und kostengünstiger produzierbaren Mehrkammerbeutel sowie ein zugehöriges Herstellungsverfahren zu schaffen.

Diese Aufgabe wird durch einen Mehrkammerbeutel mit den Merkmalen des Anspruchs 1 sowie ein Verfahren mit den Merkmalen des Anspruchs 9 gelöst.

Weitere vorteilhafte Abwandlungen der Erfindung sind Gegenstand der Unteransprüche.

Demnach ist ein Mehrkammerbeutel zur Herstellung einer medizinischen Lösung, insbesondere von Dialysat, vorgesehen, mit mindestens zwei Kammern, welche miteinander über eine Peel-Naht verbunden sind und welche jeweils an einer ersten und einer zweiten einander gegenüberliegenden Seite des Beutels von jeweils einer Siegelnaht begrenzt sind. Der Mehrkammerbeutel weist weiterhin eine durch eine umgeschlagene Folie gebildete dritte Seite auf, an welcher mindestens ein Anschluss mittels Stumpfschweißens angeordnet ist. Eine der Kammern weist keinen Anschluss auf.

Das Anbringen des Anschlusses an der dritten Seite bietet vorzugsweise den Vorteil, dass der Anschluss auf einer äußersten Schicht der umgeschlagenen Folie aufsitzt und nicht in die umgeschlagene Folie oder deren Schichtstruktur integriert ist oder zwischen zwei Folien zwischengelagert ist.

Vorzugsweise bezeichnet der Begriff Stumpfschweißen hierbei ein Verfahren, bei welchem zwei Stirnflächen in Anlage aneinander stumpf miteinander verschweißt werden. Das Stumpfschweißen ist auch unter der Bezeichnung Spiegelschweißen bekannt.

Beispielsweise weist der Anschluss einen Schlauchabschnitt auf. Zunächst wird an einer Stirnseite des Schlauchabschnitts der Schlauchabschnitt vorzugsweise tulpenförmig aufgeweitet, um eine größere Stirnfläche nach Art eines Flansches zu erzeugen. Daraufhin wird die Stirnfläche des Schlauchabschnitts auf eine Fläche, beispielsweise eine Folie, aufgesetzt und mit dieser verschweißt.

Die Stirnfläche des Schlauchabschnitts sitzt bei dieser Vorgehensweise daraufhin auf der Fläche der Folie auf und ist nicht in diese integriert, beispielsweise im Falle einer Mehrschichtfolie zwischen den Schichten der Mehrschichtfolie angeordnet oder zwischen zwei Folien zwischengelagert.

Mittels Stumpfschweißen lassen sich besonders feste und belastbare Verbindungen schaffen.

An der dritten Seite kann eine Mehrzahl an Anschlüssen angeordnet sein, von denen mindestens einer, vorzugsweise alle, mittels Stumpfschweißens an der Folie angeordnet sind.

Gemäß einer Ausführungsform weist der Mehrkammerbeutel an einer der dritten Seite gegenüberliegenden vierten Seite eine Siegelnaht mit vorzugsweise einer oder mehreren Aufhängeösen auf. Die Siegelnaht hat vorzugsweise eine derartige Breite, dass die Aufhängeöse(n) vollständig von der Siegelnaht umgrenzt ist /sind.

Vorzugsweise weist der Mehrkammerbeutel, mindestens jedoch eine Kammer des Mehrkammerbeutels, keinen Befüllport zum Befüllen mindestens einer Kammer des Mehrkammerbeutels, vorzugsweise in Form eines Anschlusses, Ports oder Konnektors, auf.

Unter einem derartigen Befüllport ist vorzugsweise ein Zugang zu einem Aufnahmebereich des Beutels zu verstehen, welcher dazu ausgelegt ist, den Aufnahmebereich des Beutels vor der Auslieferung des Beutels oder Beutelsystems an einen Endanwender bzw. vor dem Einsatz des Beutels bei der Peritonealdialyse zu befüllen. Nach der Befüllung wird der Befüllport meist unlösbar beispiels-weise mit einer Verschlusskappe verschlossen.

Ein derartiger Befüllport ist vorzugsweise nicht erforderlich, da ein erfindungsgemäßer Mehrkammerbeutel vorzugsweise mit einem erfindungsgemäßen Verfahren vor dem Verschließen des Beutels befüllt wird.

Somit ist es nicht erforderlich, über einen Port beispielsweise eine Flüssigkeit in eine Kammer des Beutels zu leiten, sondern die Kammer wird erst verschlossen und ausgebildet, nachdem sie befüllt wurde.

Denkbar ist es, dass der Mehrkammerbeutel somit nur einen Anschluss zum Entnehmen von Inhalt aus dem Beutel aufweist, wobei vorzugsweise zumindest eine Kammer des Mehrkammerbeutels über keinen eigenen Anschluss, Port oder Konnektor verfügt. Der Inhalt dieser Kammer kann vorzugsweise nur über den Anschluss einer anderen Kammer des Mehrkammerbeutels nach außen gelangen, beispielsweise nachdeme eine die Kammern trennende Peel-Naht geöffnet wurde.

Gemäß einer Ausführungsform weist der Mehrkammerbeutel eine erste Kammer und eine zweite Kammer auf, wobei die erste Kammer durch die den Anschluss aufweisende dritte Seite begrenzt wird und die zweite Kammer durch ein Öffnen der Peel-Naht mit der ersten Kammer verbindbar ist, sodass ein Inhalt der zweiten Kammer nur über die erste Kammer sowie den Anschluss an der dritten Seite des Beutels nach außen verbringbar ist.

Die zweite Kammer wird vorzugsweise ausschließlich durch die Peel-Naht, die Siegelnähte an der ersten und zweiten Seite sowie die Siegelnaht an der vierten Seite begrenzt.

Vorzugsweise erstreckt sich eine Längsachse der Peel-Naht rechtwinklig oder im Wesentlichen rechtwinklig zu einer Längsachse des Mehrkammerbeutels. So lassen sich auf einfache Weise zwei Kammern unterschiedlicher Größe ausbilden.

Alternativ kann sich die Peel-Naht auch parallel zu einer Längsachse des Mehrkammerbeutels erstrecken.

Gemäß einer Ausführungsform weisen die mindestens zwei Kammern des Beutels jeweils einen Inhalt auf, welcher dazu ausgelegt ist, bei einer Vereinigung des Inhalts mit einem Inhalt der anderen Kammer des Mehrkammerbeutels gebrauchsfertiges Dialysat zu ergeben.

Beispielsweise kann mindestens eine Kammer ein saures Konzentrat und die andere Kammer eine ionische Lösung enthalten. Mindestens eine Kammer kann Bikarbonat und / oder ein Osmotikum, beispielsweise ein Sachharid, enthalten.

Weiterhin betrifft die vorliegende Erfindung ein Verfahren zum Herstellen eines Mehrkammerbeutels, vorzugsweise eines erfindungsgemäßen Mehrkammerbeutels, mit den Schritten:
a) Vorsehen einer Flachfolie mit zwei Außenkanten; wobei die Flachfolie eine Mehrschicht- oder Einschichtfolie sein kann,
b) Fixieren mindestens eines Anschlusses an der Flachfolie mittels Stumpfschweißens, und vorzugsweise Erzeugen einer Öffnung der Flachfolie im Bereich des Anschlusses, beispielsweise durch Ausstanzen oder mittels eines heißen Dorns, wobei der Anschluss an einer durch Umschlagen der Flachfolie gebildeten dritten Seite des Mehrkammerbeutels angeordnet wird,
c) Erzeugen eines Folienschlauchs aus der Flachfolie vorzugsweise mittels einer Formschulter, über welche die Flachfolie läuft, und Versiegeln der Außenkanten der Flachfolie miteinander,
d) Erzeugen einer Peel-Naht zwischen zwei Lagen der Flachfolie des Folienschlauchs, welche die zwei Lagen der Flchfolie lösbar miteinder verbindet, zwischen zwei Außenkanten des Folienschlauchs entlang einer Längsrichtung des Folienschlauchs verläuft, wodurch mindestens zwei mittels der Peel-Naht voneinander getrennte Aufnahmebereiche entstehen;
e) Erzeugen einer rechtwinklig oder im Wesentlichen rechtwinklig zu der Peel-Naht verlaufenden Siegelnaht, wodurch in den zwei Aufnahmebereichen jeweils eine Aufnahmetasche entsteht,
f) Befüllen der zwei Aufnahmetaschen entlang der Längsrichtung des Folienschlauchs vorzugsweise mit zwei unterschiedlichen Inhalten; und
g) Verschließen der zwei Aufnahmetaschen durch ein Erzeugen einer weiteren rechtwinklig oder im Wesentlichen rechtwinklig zu der Peel-Naht verlaufenden Siegelnaht, wodurch die Aufnahmetaschen verschlossen werden und mindestens zwei vollständig verschlossene Kammern ausgebildet werden, von denen eine keinen Anschluss aufweist

Im Rahmen des Stumpfschweißens in Schritt b) kommen vorzugsweise die folgenden Parameter zum Einsatz:

| | | | | | |
|---|---|---|---|---|---|
| Schweißtemperatur: | 150°C | ≤ | T | ≤ | 300°C |
| Schweißzeit: | 0,7 s | ≤ | t | ≤ | 2 s |
| Schweißdruck: | 0,1 N/mm² | ≤ | *σ* | ≤ | 2 N/mm² |

Besonders bevorzugt sind die nachstehenden Parameter:

| | | | | | |
|---|---|---|---|---|---|
| Schweißtemperatur: | 190°C | ≤ | T | ≤ | 250°C |
| Schweißzeit: | 0,9 s | ≤ | t | ≤ | 1,5 s |

Schweißdruck: 3000N bei A=6300mm² → 0,48N/mm²

Vorzugsweise werden kontinuierlich entlang eines fortlaufenden Folienschlauchs jeweils über eine Siegelnaht miteinander verbundene Mehrkammerbeutel gefertigt, welche jeweils durch ein Trennen entlang einer rechtwinklig oder im Wesentlichen rechtwinklig zu der Peel-Naht verlaufenden Siegelnaht voneinander separiert werden.

In anderen Worten wird vorzugsweise ein durchgängiger Folienschlauch gefertigt, welcher in Längsrichtung des Schlauchs befüllt wird und welcher durch Siegeln in Querrichtung in unterschiedliche Beutel portioniert wird.

Somit entstehen beim Befüllen des Schlauches vorzugsweise zwei parallel zueinander und über die Peel-Naht getrennte Säulen an Füllgut, welche durch Quersiegeln in mehrere Mehrkammerbeutel aufteilt werden.

Gemäß einer Ausführungsform werden in Schritt f) die mindestens zwei Aufnahmetaschen von zwei separaten Befülllanzen mit einem flüssigen, festen und / oder pastösen Inhalt befüllt, welcher vorzugsweise für die Herstellung von Dialysat ausgelegt ist.

Vorzugsweise ist ein erfindungsgemäßer Mehrkammerbeutel sowie dessen stumpfgeschweißter Anschluss aus einem Polymermaterial gefertigt und ein Einmal-Artikel oder Disposable ausgebildet.

Vorzugsweise wird ein erfindungsgemäßes Verfahren mit einer einzigen Maschine ausgeführt.

An dieser Stelle ist darauf hinzuweisen, dass die die vorliegende Offenbarung nicht auf die explizit genannten Merkmalskombinationen beschränkt ist, sondern die hierin offenbarten Merkmale auch in anderen Kombinationen oder in Isolation beansprucht werden können.

Weitere Vorteile, Effekte und Merkmale der vorliegenden Erfindung ergeben sich aus der nachstehenden Beschreibung bevorzugter Ausführungsformen unter Bezugnahme auf die Zeichnungen, in welchen gleiche Bezugszeichen gleiche oder ähnliche Bauteile bezeichnen.

Hierbei zeigt:
Fig. 1 einen erfindungsgemäßen Mehrkammerbeutel, und
Fig. 2 eine kontinuierliche Herstellung erfindungsgemäßer Mehrkammerbeutel aus einem durchgängigen Folienschlauch.

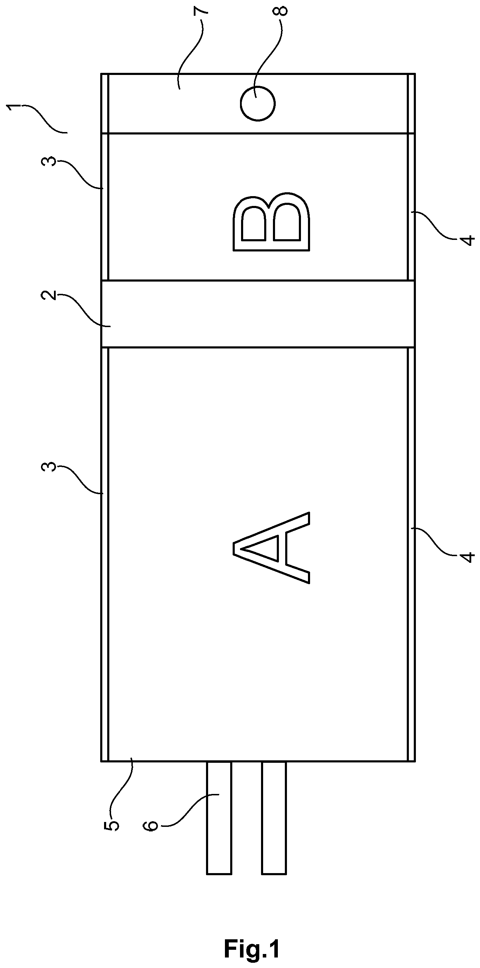

Fig. 1 zeigt einen erfindungsgemäßen Mehrkammerbeutel 1 mit zwei Kammern A und B, welche miteinander über eine Peel-Naht 2 verbunden sind und welche jeweils an einer ersten und einer zweiten einander gegenüberliegenden Seite 3 und 4 von jeweils einer Siegelnaht begrenzt sind.

Der Mehrkammerbeutel 1 weist weiterhin eine durch eine umgeschlagene Folie gebildete dritte Seite 5 auf, an welcher zwei Anschlüsse in Form von Schlauchabschnitten 6 mittels Stumpfschweißen angeordnet sind.

Der Beutel 1 wird aus einer Flachfolie durch Umschlagen der Folie an der dritten Seite 5 und Miteinanderversiegeln der Außenkanten der Flachfolie an einer vierten Seite zu einer Siegelnaht 7 erzeugt, wodurch zunächst ein Folienschlauch entsteht. In der Siegelnaht 7 ist mindestens eine Aufhängeöse 8 angebracht, welche den Anschlüssen 6 gegenüberliegt.

Daraufhin wird die in Fig. 1 am unteren Ende (zweite Seite 4) des Beutels 1 befindliche Quersiegelnaht erzeugt.

Die Kammern A und B sind in diesem Zwischenzustand an ihrer in Fig. 1 oberen Seite noch offen und bilden somit Aufnahmetaschen aus, welche bequem von oben (in der in Fig. 1 gezeigten Ansicht) befüllt werden können.

Nach dem Befüllen der Aufnahmetaschen wird die in Fig. 1 am oberen Ende (erste Seite 3) des Beutels 1 befindliche Quersiegelnaht erzeugt, wodurch die Aufnahmetaschen verschlossen werden und die Kammern A und B ausgebildet werden.

Die Kammer B weist keinen eigenen Anschluss oder Port auf, sodass der Inhalt der Kammer B nur über die geöffnete Peel-Naht 2, die Kammer A und deren Anschlüsse nach außen gelangen kann.

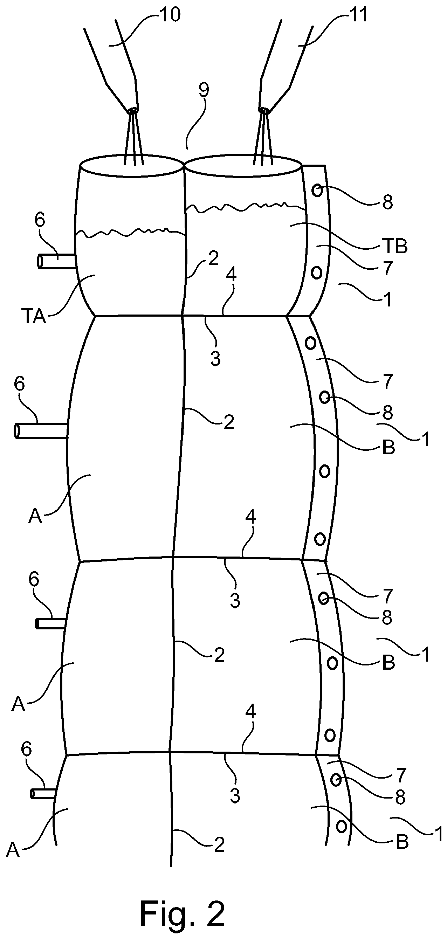

Fig. 2 zeigt eine kontinuierliche Herstellung erfindungsgemäßer Mehrkammerbeutel 1 aus einem durchgängigen Folienschlauch 9.

Wie in Fig. 2 gezeigt, erstreckt sich ein Folienschlauch 9 während des Befüllens vorzugsweise in Vertikalrichtung. In der in Fig. 2 gezeigten Ansicht umfasst der Folienschlauch 9 vier Mehrkammerbeutel 1, welche in dieser Ansicht noch nicht voneinander separiert sind, sondern jeweils durch eine gemeinsame Quersiegelnaht an den Seiten 3,4 miteinander verbunden sind.

Die gemeinsame Quersiegelnaht an den Seiten 3,4 trennt die Kammern A und B benachbarter Mehrkammerbeutel 1. Jeder Beutel 1 ist mit einem stumpfgeschweißten Anschluss 6 ausgestattet.

Der in Fig.2 gezeigte oberste Beutel verdeutlicht die Befüllung der Kammern A und B. Bei diesem Beutel sind die Kammern A und B an ihrem in Fig. 2 gezeigten oberen Ende noch nicht verschlossen und bilden daher Aufnahmetaschen TA und TB für Füllgut aus.

Eine jede Aufnahmetasche TA, TB wird mittels einer eigenen Befüllvorrichtung, z.B. einer Befülllanze 10,11 vorzugsweise von oben in Vertikalrichtung mit einem bestimmten Inhalt beschickt. Sind die Aufnahmetaschen TA und TB ausreichend gefüllt, werden sie durch Quersiegeln verschlossen.

Durch diese Art der Befüllung entfällt die Notwendigkeit, die Kammern A und B des Mehrkammerbeutels jeweils mit einem Befüllanschluss oder Port auszustatten.

Um die einzelnen Beutel 1 des kontinuierlichen Folienschlauchs 9 voneinander zu trennen, werden sie in Querrichtung entlang der gemeinsamen Quersiegelnaht an den Seiten 3,4 auseinandergeschnitten.

## Patentansprüche

1. Mehrkammerbeutel (1) zur Herstellung einer medizinischen Lösung, insbesondere von Dialysat, mit mindestens zwei Kammern (A, B), welche miteinander über eine Peel-Naht (2) verbunden sind und welche jeweils an einer ersten und einer zweiten einander gegenüberliegenden Seite von jeweils einer Siegelnaht begrenzt sind, **dadurch gekennzeichnet, dass** der Mehrkammerbeutel weiterhin eine durch eine umgeschlagene Folie gebildete dritte Seite (5) aufweist, an welcher mindestens ein Anschluss (6) mittels Stumpfschweißens angeordnet ist und dass eine Kammer (B) keinen Anschluss aufweist.

2. Mehrkammerbeutel (1) gemäß Anspruch 1, **dadurch gekennzeichnet, dass** der Mehrkammerbeutel (1) an einer der dritten Seite (5) gegenüberliegenden vierten Seite eine Siegelnaht (7) mit vorzugsweise einer Aufhängeöse (8) aufweist.

3. Mehrkammerbeutel (1) gemäß Ansprüche 1 oder 2, **dadurch gekennzeichnet, dass** der Mehrkammerbeutel (1) keinen Befüllport zum Befüllen mindestens einer Kammer des Mehrkammerbeutels (1), vorzugsweise in Form eines Anschlusses, Ports oder Konnektors, aufweist.

4. Mehrkammerbeutel (1) gemäß einem der vorhergehenden Ansprüche, **dadurch gekennzeichnet, dass** der Mehrkammerbeutel (1) eine erste Kammer (A) und eine zweite Kammer (B) aufweist, wobei die erste Kammer (A) zumindest teilweise durch die den Anschluss (6) aufweisende dritte Seite (5) begrenzt wird und die zweite Kammer (B) durch ein Öffnen der Peel-Naht (2) mit der ersten Kammer (A) verbindbar ist, sodass ein Inhalt der zweiten Kammer (B) nur über die erste Kammer (A) sowie den Anschluss (6) an der dritten Seite (5) nach außen verbringbar ist.

5. Mehrkammerbeutel (1) Anspruch 4, **dadurch gekennzeichnet, dass** die zweite Kammer (B) ausschließlich durch die Peel-Naht (2), die Siegelnähte an der ersten und zweiten Seite sowie die Siegelnaht (7) an der vierten Seite begrenzt wird.

6. Mehrkammerbeutel (1) gemäß einem der vorhergehenden Ansprüche, **dadurch gekennzeichnet, dass** sich eine Längsachse der Peel-Naht (2) rechtwinklig oder im Wesentlichen rechtwinklig zu einer Längsachse des Mehrkammerbeutels (1) erstreckt.

7. Mehrkammerbeutel (1) gemäß einem der vorhergehenden Ansprüche, **dadurch gekennzeichnet, dass** die mindestens zwei Kammern (A, B) des Beutels (1) jeweils einen Inhalt aufweisen, welcher dazu ausgelegt ist, bei einer Vereinigung des Inhalts mit einem Inhalt der anderen Kammer des Mehrkammerbeutels (1) gebrauchsfertiges Dialysat zu ergeben.

8. Mehrkammerbeutel (1) gemäß einem der vorhergehenden Ansprüche, **dadurch gekennzeichnet, dass** der Anschluss (6) an der dritten Seite (5) derart angeschweißt ist, dass er auf einer äußersten Schicht der umgeschlagenen Folie aufsitzt und nicht zwischen zwei Folien angeordnet ist oder in die umgeschlagene Folie oder deren Schichtstruktur integriert ist.

9. Verfahren zum Herstellen eines Mehrkammerbeutels (1) zur Herstellung einer medizinischen Lösung, vorzugsweise eines Mehrkammerbeutels (1) gemäß einem der Ansprüche 1 bis 8, mit den Schritten:
a) Vorsehen einer Flachfolie mit zwei Außenkanten;
b) Fixieren mindestens eines Anschlusses (6) an der Fachfolie mittels Stumpfschweißens, wobei der Anschluss (6) an einer durch Umschlagen der Flachfolie gebildeten dritten Seite (5) des Mehrkammerbeutels (1) angeordnet wird,
c) Erzeugen eines Folienschlauchs (9) aus der Flachfolie durch Versiegeln der Außenkanten der Flachfolie miteinander,
d) Erzeugen einer Peel-Naht (2), welche zwischen zwei Außenkanten des Folienschlauchs (9) entlang einer Längsrichtung des Folienschlauchs verläuft, wodurch mindestens zwei mittels der Peel-Naht (2) voneinander getrennte Aufnahmebereiche (A, B) entstehen;
e) Erzeugen einer rechtwinklig oder im Wesentlichen rechtwinklig zu der Peel-Naht (2) verlaufenden Siegelnaht, wodurch in den zwei Aufnahmebereichen jeweils eine Aufnahmetasche (TA, TB) entsteht,
f) Befüllen der zwei Aufnahmetaschen (TA, TB) entlang der Längsrichtung des Folienschlauchs (9) vorzugsweise mit zwei unterschiedlichen Inhalten; und
g) Verschließen der zwei Aufnahmetaschen (TA, TB) durch ein Erzeugen einer weiteren rechtwinklig oder im Wesentlichen rechtwinklig zu der Peel-Naht (2) verlaufenden Siegelnaht, wodurch die Aufnahmetaschen (TA, TB)verschlossen werden und mindestens zwei vollständig verschlossene Kammern (A, B) ausgebildet werden, von denen eine Kammer (B) keinen Anschluss aufweist.

10. Verfahren gemäß Anspruch 9, **dadurch gekennzeichnet, dass** kontinuierlich entlang eines fortlaufenden Folienschlauchs (9) jeweils über eine Siegelnaht miteinander verbundene Mehrkammerbeutel (1) gefertigt werden, welche jeweils durch ein Trennen entlang einer rechtwinklig oder im Wesentlichen rechtwinklig zu der Peel-Naht (2) verlaufenden Siegelnaht voneinander separiert werden.

11. Verfahren gemäß Anspruch 8 oder 9, **dadurch gekennzeichnet, dass** in Schritt f) die zwei Aufnahmetaschen (TA, TB) von zwei separaten Befülllanzen (10, 11) mit einem flüssigen, festen und / oder pastösen Inhalt befüllt werden, welcher für die Herstellung von Dialysat ausgelegt ist.

## Claims

1. A multi-chamber bag (1) for producing a medical solution, in particular dialysate, comprising at least two chambers (A, B), which are connected to one another via a peel seam (2) and which are each delimited on a first and a second mutually opposite side by a respective sealed seam, **characterized in that the multi-chamber bag** further comprises a third side (5) formed by a folded-over film, on which at least one port (6) is arranged by means of butt welding, and that one chamber (B) comprises no port.

2. The multi-chamber bag (1) according to claim 1, **characterized in that** the multi-chamber bag (1) comprises on a fourth side opposite the third side (5) a sealed seam (7) with preferably a suspension eyelet (8).

3. The multi-chamber bag (1) according to claims 1 or 2, **characterized in that** the multi-chamber bag (1) comprises no filling port for filling at least one chamber of the multi-chamber bag (1), preferably in the form of a port or connector.

4. The multi-chamber bag (1) according to any one of the preceding claims, **characterized in that** the multi-chamber bag (1) comprises a first chamber (A) and a second chamber (B), wherein the first chamber (A) is at least partially delimited by the third side (5) comprising the port (6), and the second chamber (B) is connectable to the first chamber (A) by opening the peel seam (2), such that a content of the second chamber (B) can be transferred to the outside only via the first chamber (A) and the port (6) on the third side (5).

5. The multi-chamber bag (1) claim 4, **characterized in that** the second chamber (B) is delimited exclusively by the peel seam (2), the sealed seams on the first and second sides, and the sealed seam (7) on the fourth side.

6. The multi-chamber bag (1) according to any one of the preceding claims, **characterized in that** a longitudinal axis of the peel seam (2) extends at a right angle or essentially at a right angle to a longitudinal axis of the multi-chamber bag (1).

7. The multi-chamber bag (1) according to any one of the preceding claims, **characterized in that** the at least two chambers (A, B) of the bag (1) each comprise a content which is configured to yield ready-to-use dialysate upon combination of the content with a content of the other chamber of the multi-chamber bag (1).

8. The multi-chamber bag (1) according to any one of the preceding claims, **characterized in that** the port (6) is welded on the third side (5) in such a way that it rests on an outermost layer of the folded-over film and is not arranged between two films or integrated into the folded-over film or its layer structure.

9. A method for producing a multi-chamber bag (1) for producing a medical solution, preferably a multi-chamber bag (1) according to any one of claims 1 to 8, with the steps:
a) providing a flat film with two outer edges;
b) fixing at least one port (6) on the flat film by means of butt welding, wherein the port (6) is arranged on a third side (5) of the multi-chamber bag (1) formed by folding over the flat film,
c) creating a film tube (9) from the flat film by sealing the outer edges of the flat film to one another,
d) creating a peel seam (2) which runs between two outer edges of the film tube (9) along a longitudinal direction of the film tube, whereby at least two receiving areas (A, B) separated from one another by means of the peel seam (2) are created;
e) creating a sealed seam running at a right angle or essentially at a right angle to the peel seam (2), whereby a receiving pocket (TA, TB) is created in each of the two receiving areas,
f) filling the two receiving pockets (TA, TB) along the longitudinal direction of the film tube (9) preferably with two different contents; and
g) closing the two receiving pockets (TA, TB) by creating a further sealed seam running at a right angle or essentially at a right angle to the peel seam (2), whereby the receiving pockets (TA, TB) are closed and at least two completely closed chambers (A, B) are formed, of which one chamber (B) comprises no port.

10. The method according to claim 9, **characterized in that** multi-chamber bags (1) which are each connected to one another via a sealed seam are manufactured continuously along a continuous film tube (9), which are each separated from one another by a separating along a sealed seam running at a right angle or essentially at a right angle to the peel seam (2).

11. The method according to claim 8 or 9, **characterized in that** in step f) the two receiving pockets (TA, TB) are filled by two separate filling lances (10, 11) with a liquid, solid and/or pasty content which is configured for the production of dialysate.

## Revendications

1. Poche à plusieurs compartiments (1) pour la production d'une solution médicale, en particulier de dialysat, comprenant au moins deux compartiments (A, B), lesquels sont reliés l'un à l'autre par une soudure pelable (2) et lesquels sont délimités chacun sur un premier et un deuxième côté opposés l'un à l'autre par respectivement une soudure d'étanchéité,
**caractérisée en ce que** la poche à plusieurs compartiments présente en outre un troisième côté (5) formé par une feuille repliée, sur lequel au moins un raccord (6) est agencé au moyen d'un soudage bout à bout et **en ce qu'**un compartiment (B) ne présente aucun raccord.

2. Poche à plusieurs compartiments (1) selon la revendication 1, **caractérisée en ce que** la poche à plusieurs compartiments (1) présente, sur un quatrième côté opposé au troisième côté (5), une soudure d'étanchéité (7) avec de préférence un œillet de suspension (8).

3. Poche à plusieurs compartiments (1) selon les revendications 1 ou
2, **caractérisée en ce que** la poche à plusieurs compartiments (1) ne présente aucun orifice de remplissage pour le remplissage d'au moins un compartiment de la poche à plusieurs compartiments (1), de préférence sous la forme d'un raccord, d'un orifice ou d'un connecteur.

4. Poche à plusieurs compartiments (1) selon l'une quelconque des revendications précédentes, **caractérisée en ce que** la poche à plusieurs compartiments (1) présente un premier compartiment (A) et un deuxième compartiment (B), dans laquelle le premier compartiment (A) est au moins partiellement délimité par le troisième côté (5) présentant le raccord (6) et le deuxième compartiment (B) peut être relié au premier compartiment (A) par une ouverture de la soudure pelable (2), de sorte qu'un contenu du deuxième compartiment (B) ne peut être évacué vers l'extérieur que par l'intermédiaire du premier compartiment (A) ainsi que du raccord (6) sur le troisième côté (5).

5. Poche à plusieurs compartiments (1) revendication 4, **caractérisée en ce que** le deuxième compartiment (B) est exclusivement délimité par la soudure pelable (2), les soudures d'étanchéité sur le premier et le deuxième côté ainsi que la soudure d'étanchéité (7) sur le quatrième côté.

6. Poche à plusieurs compartiments (1) selon l'une quelconque des revendications précédentes, **caractérisée en ce que** un axe longitudinal de la soudure pelable (2) s'étend perpendiculairement ou essentiellement perpendiculairement à un axe longitudinal de la poche à plusieurs compartiments (1).

7. Poche à plusieurs compartiments (1) selon l'une quelconque des revendications précédentes, **caractérisée en ce que** les au moins deux compartiments (A, B) de la poche (1) présentent chacun un contenu qui est conçu pour donner, lors d'une union du contenu avec un contenu de l'autre compartiment de la poche à plusieurs compartiments (1), du dialysat prêt à l'emploi.

8. Poche à plusieurs compartiments (1) selon l'une quelconque des revendications précédentes, **caractérisée en ce que** le raccord (6) est soudé sur le troisième côté (5) de telle sorte qu'il repose sur une couche la plus externe de la feuille repliée et n'est pas agencé entre deux feuilles ou intégré dans la feuille repliée ou sa structure en couches.

9. Procédé de production d'une poche à plusieurs compartiments (1) pour la production d'une solution médicale, de préférence d'une poche à plusieurs compartiments (1) selon l'une quelconque des revendications 1 à 8, avec les étapes :
a) fourniture d'une feuille plate avec deux bords extérieurs ;
b) fixation d'au moins un raccord (6) sur la feuille plate au moyen d'un soudage bout à bout, dans lequel le raccord (6) est agencé sur un troisième côté (5) de la poche à plusieurs compartiments (1) formé par repliage de la feuille plate,
c) création d'une gaine de feuille (9) à partir de la feuille plate par scellage des bords extérieurs de la feuille plate l'un avec l'autre,
d) création d'une soudure pelable (2), laquelle s'étend entre deux bords extérieurs de la gaine de feuille (9) le long d'une direction longitudinale de la gaine de feuille, moyennant quoi au moins deux zones de réception (A, B) séparées l'une de l'autre au moyen de la soudure pelable (2) sont produites ;
e) création d'une soudure d'étanchéité s'étendant perpendiculairement ou essentiellement perpendiculairement à la soudure pelable (2), moyennant quoi une poche de réception (TA, TB) est produite respectivement dans les deux zones de réception,
f) remplissage des deux poches de réception (TA, TB) le long de la direction longitudinale de la gaine de feuille (9) de préférence avec deux contenus différents ; et
g) fermeture des deux poches de réception (TA, TB) par une création d'une autre soudure d'étanchéité s'étendant perpendiculairement ou essentiellement perpendiculairement à la soudure pelable (2), moyennant quoi les poches de réception (TA, TB) sont fermées et au moins deux compartiments (A, B) entièrement fermés sont formés, dont un compartiment (B) ne présente aucun raccord.

10. Procédé selon la revendication 9, **caractérisé en ce que** des poches à plusieurs compartiments (1) reliées les unes aux autres respectivement par une soudure d'étanchéité sont fabriquées en continu le long d'une gaine de feuille continue (9), lesquelles sont séparées les unes des autres respectivement par une séparation le long d'une soudure d'étanchéité s'étendant perpendiculairement ou essentiellement perpendiculairement à la soudure pelable (2).

11. Procédé selon la revendication 8 ou 9, **caractérisé en ce que**, à l'étape f), les deux poches de réception (TA, TB) sont remplies par deux lances de remplissage séparées (10, 11) avec un contenu liquide, solide et/ou pâteux, lequel est conçu pour la production de dialysat.
